# EUROPEAN PATENT APPLICATION

(11) **EP 1 591 440 A1**
(43) Date of publication of application: **02.11.2005**
(21) Application number: 03815509.9
(22) Date of filing: 28.01.2003
(51) Int. Cl.: C07C 229/36, C07C 227/14, A61K 31/198, A61K 31/221

(54) **ALANINES COMPOUNDS, METHOD OF PREPARING THEM AND THEIR USE**

(71) Applicant: SHANGHAI INSTITUTE OF MATERIA MEDICA, CHINESE ACADEMY OF SCIENCES, Shanghai 200031 (CN)
(72) Inventor: YANG, Yushe Shanghai Institute of Materia Medica, Shanghai 200031 (CN); TANG, Lei Shanghai Institute of Materia Medica, Shanghai 200031 (CN); JI, Ruyun Shanghai Institute of Materia Medica, Shanghai 200031 (CN); CHEN, Kaixian Shanghai Inst. of Materia Medical, Shanghai 200031 (CN)
(74) Representative: Rupprecht, Kay
(86) International application number: PCT/CN2003/000096
(87) International publication number: WO 2004/067495

(57) **Abstract**

The present invention provides alanine compounds of formula (I) or their salts:

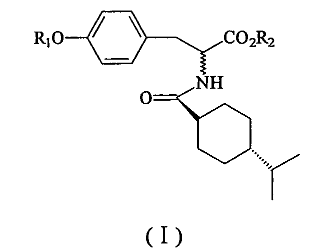

In the formula (I), the configuration of α ― Carbon atom of alanine is R or S; R₁ is hydrogen, unsubstituted or substituted C₁₋₆ alkyl, unsubstituted or substituted aromatic ring or aromatic heterocyclic ring; R₂ is hydrogen or unsubstituted or substituted C₁₋₆ alkyl. The present invention also provides two methods of preparing these compounds or their salts, and their use in preparing anti II type diabetic medicine.

## Description

### Fields of invention

The present invention relates to the fields of medicinal chemistry and endocrinotherapy, specifically to the synthesis of alanine compounds and their use in the preparation of drugs for type II diabetes.

### Background Art

Type II diabetes is a metabolic disorder characterized by hyperglycemia (the fasting blood glucose concentration is above 130mg/dL) and glucosuria. Long-term hyperglycemia often leads to various complications, such as neuropathy, retinopathy and nephropathy. Especially, the cardiovascular complications are the main causes of leading to disability and death in diabetic patients [Shinkai, H. Exp. Opin. Ther. Patents. 2000, 10: 596]. Therefore, controlling the blood glucose level in diabetic patients is very important for postponing or blocking the occurrence of the complications. Drugs currently available for clinically controlling the blood glucose level in the patients are mainly sulfonylureas for promoting the release of insulin and biguanides. Since insulin resistance is the predominant pathogenesis in type II diabetes, study on insulin-sensitizing agents has become an important direction in the development of anti-type II diabetes drugs. The first thiazolidinedione insulin-sensitizer troglitazone was marketed in 1997. This drug and another two thiazolidinediones, piglitazone and rosiglitazone which were released later, were reported to clinically control the blood glucose level in patients effectively. However, hepatic toxicity were found for these thiazolidinediones after marketing [Henry, R. R. Endocrinol. Metab. Clic. North Am. 1997, 26, 553], and troglitazone was even withdrawn from market for its higher hepatic toxicity. The toxicity of this type of compounds was suspected relating to the thiazolidinedione ring group, so the emphasis of insulin-sensitizers study has transferred to the synthesis and development of non-thiazolidinedione compounds become gradually a main trend of the study on the anti-type II diabetes drugs.

The references mentioned above are cited herein in their entireties.

One objective of the present invention is to provide novel alanine compounds having insulin-sensitizing activities and their pharmaceutical acceptable salts.

Another objective of the present invention is to provide preparative methods for the alanine compounds and their salts.

A further objective of the present invention is to provide the application of the alanine compounds and their salts in the preparation of drugs for type II diabetes.

### Summary of the Invention

The invention provides the alanine compounds represented by the following formula (I) and their salts:

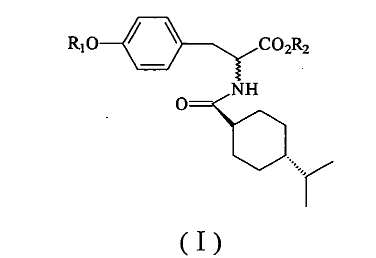

In the formula (I), the configuration of α -carbon atom of alanine is R or S.

R₁ is hydrogen, unsubstituted or substituted C₁₋₆ alkyl, or unsubstituted or substituted aromatic ring group or aromatic heterocyclic group, such as

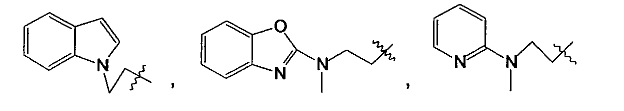

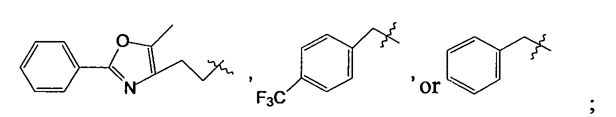

R₂ is hydrogen or unsubstituted or substituted C₁₋₆ alkyl.

The present invention also provides two preparative methods for the alanine compounds with the structure of formula (I) and their salts.

The first preparative method includes the following steps:
(1) Condensing *trans*-4-isopropylcyclohexylcarboxylic acid N-hydroxylsuccinimide ester (compound A) and L-tyrosine methyl ester or D-tyrosine methyl ester in an inert solvent to obtain 2-[N-(*trans*-4-isopropylcyclohexylcarbonyl)amino]-3-(4-hydroxyphenyl)propionic acid methyl ester (compound B);
(2) Conducting Mitsunobu reaction of Compound B and corresponding heterocycloalkyl alcohol or aromatic alcohol, followed by hydrolyzation with inorganic bases to obtain the compound of formula (I), wherein R₁ is: 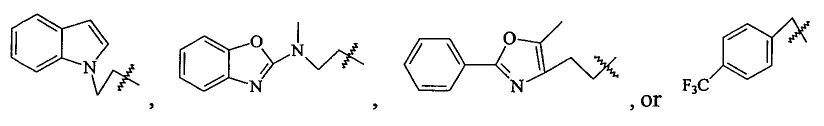 R₂ is H; or
   Conducting an etherification of compound B and corresponding alkyl halide under alkali condition to obtain the compound of formula (I), wherein R₁ is: 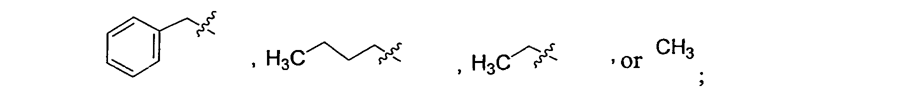 R₂ is H; or
   Hydrolyzing compound B to obtain the compound of formula (I), wherein R₁ and R₂ both are hydrogen;
(3) preparing the corresponding pharmaceutical acceptable salts according to requirement.

The second preparative method includes the following steps:
(1) Condensing compound B with amino-protected 2-methylaminoethanol, then deprotecting, and reacting with excessive 2-fluoropyridine under reflux, followed by hydrolysis under basic conditions to give compounds of formula (I), wherein R₁ is 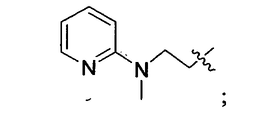 R₂ is hydrogen;
(2) corresponding pharmaceutical acceptable salts are prepared according to requirement.

The present invention further provides application of alanine compounds of formula (I) and their salts in the preparation of drugs for type II diabetes.

### Description of the Invention

Unless otherwise specified, the terms in the description have the following definitions:

The "C₁₋₆ alkyl" represents the saturated or unsaturated, substituted or unsubstituted linear or branched alkane-derived radical containing 1 to 6 carbon atoms, the specific examples include methyl, ethyl, propyl, isopropyl, butyl, isobutyl, *sec*-butyl, *tert*-butyl, amyl, isoamyl, *neo*-pentyl, *tert*-amyl, 1-methylbutyl, 2-methylpropyl, hexyl, isohexyl, 1-methylamyl, 2-methylamyl, 3-methylamyl, 2-methylbutyl, 1,1-dimethylbutyl, 1,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl or the like. Among these groups, the alkyls of 1 to 4 carbon atoms, such as methyl, ethyl, propyl, isopropyl, butyl, or the like, are preferable. And methyl, ethyl, propyl are more preferable, methyl and ethyl are the most preferable.

The "aryl" means aromatic radical, and the aryl of 6 to 14 carbon atoms are preferable, the specific examples include phenyl, tolyl, xylyl, biphenyl, naphthyl, indenyl, anthryl, phenanthryl, phenyl and naphthyl are more preferable, and phenyl is the most preferable.

The "aromatic heterocyclic group" means five or six-membered hetero aromatic radical containing 1 to 4 hetero atoms selected from oxygen atom, nitrogen atom or sulfur atom, such as furyl, thienyl, pyrrolyl, imidazolyl, thiazolyl, pyrazolyl, isothiazolyl, isooxazolyl, pyridyl, pyrimidinyl, pyridazinyl, pyrazinyl, triazolyl, tetrazolyl , or the like. Among these groups, thienyl, furyl, oxazolyl, isooxazolyl and thiazolyl are preferable, thienyl, oxazolyl and isooxazolyl are more preferable.

The "substituted alkyl", "substituted aryl" and "substituted aromatic heterocyclic group" mean the above "alkyl", "aryl" and "heteroaryl" can be optionally substituted by the groups selected from halogen atoms, alkyl, alkoxyl, acyloxy, -OH, -NH₂, -NO₂, -NHAc.

The "pharmaceutical acceptable salts" may specifically include the salts with inorganic acids, such as hydrochloric acid, hydrobromic acid, hydrofluoric acid, sulfuric acid, nitric acid, phosphoric acid or the like; the acid-addtion salts with organic acids, such as formic acid, acetic acid, propionic acid, oxalic acid, malonic acid, succinic acid, fumaric acid, maleic acid, lactic acid, malic acid, tartaric acid, citric acid, picric acid, methanesulfonic acid, ethylsulfonic acid or the like, or with acidic amino acids, such as aspartic acid, glutamic acid or the like; or the salts formed with alkalis, such as the salts with inorganic alkalis of Na, K, Ca, Al or the like, ammonium salt, methylamine salt, ethylamine salt, ethanolamine salt or the like; or the salts formed with basic amino acids, such as lysine, arginine, ornithine or the like.

The alanine compounds of formula (I) and their salts in the present invention are prepared as follows:

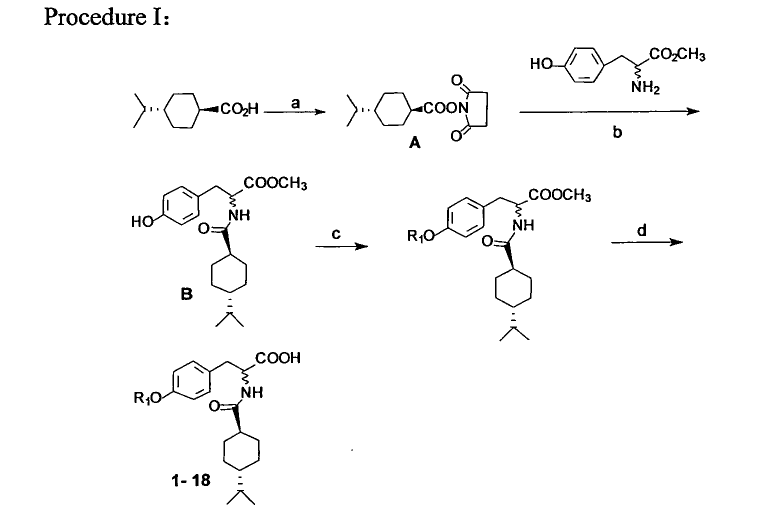

In the scheme: a. N-hydroxylsuccimide, Dicyclohexylcarbodiimide; b. chloroform; c. R₃-OH, triphenylphosphine, diethyl azodicarboxylate; or RX, K₂CO₃, DMF; d. 1N lithium hydroxide, tetrahydrofuran/methanol (3:1).

An embodiment of procedure I is as follows:
1. *trans*-4-isopropylcyclohexylcarboxylic acid (Shinkai H. J. Med. Chem. 1989, 32, 1436-1441) reacts with N-hydroxysuccimide (HOSu) and N,N'-Dicyclohexylcarbodiimide (DCC) to dehydrate and obtain *trans*-4-isopropylcyclohexylcarboxylic acid N-hydroxysuccinimide ester (compound A). The compound A condense with L-tyrosine methyl ester or D-tyrosine methyl ester in an inert solvent such as chloroform, dichloromethane, ether, tetrahydrofuran under a temperature of -10 °C -50 °C for 0.1-72h to obtain 2-[N-(*trans*-4-isopropylcyclohexylcarbonyl)amino]-3-(4-hydroxyphenyl)propionic acid methyl ester (compound B). The optimal reaction condition is to react in chloroform under room temperature for 24h.
   Compound B and corresponding heterocycloalkyl alcohol or aromatic alcohol conduct the Mitsunobu reaction, then is hydrolyzed with inorganic base to give compounds 1-8. The solvents used in the Mitsunobu reaction are the anhydrous inert solvents, such as anhydrous tetrahydrofuran, anhydrous ether, chloroform, dichloromethane or the like. The reaction temperature is between 0-100°C, the reaction time is between 0.1-3 days. The inorganic bases suitable for hydrolyzation are sodium hydroxide, lithium hydroxide, potassium hydroxide, sodium carbonate, potassium carbonate, lithium carbonate, etc; the hydrolyzing temperatures are between -10-100°C; the solvent is a mixture of tetrahydrofuran and methanol in different ratios, or a mixture of other inert solvents, such as chloroform, dichloromethane, benzene, and suitable alcohols, such as ethanol, propanol, isopropanol in different ratios. The optimal hydrolyzing condition is to hydrolyze with lithium hydroxide under room temperature for 24h with tetrahydrofuran/ methanol (3:1)) as solvent.
2. Compound B conduct etherification with corresponding alkyl halide under basic conditions to obtain compounds 9-16. The suitable inorganic bases for etherification reaction are sodium hydroxide, lithium hydroxide, potassium hydroxide, sodium carbonate, potassium carbonate, lithium carbonate, etc. The etherification temperature is between -10-180°C, The suitable solvents are dimethylformamide, DMSO, H₂O, etc. The reaction time is 1-72h.
3. Compounds 17-18 are obtained directly by hydrolyzation of compound B.
4. Corresponding pharmaceutical acceptable salts are prepared according to requirement.

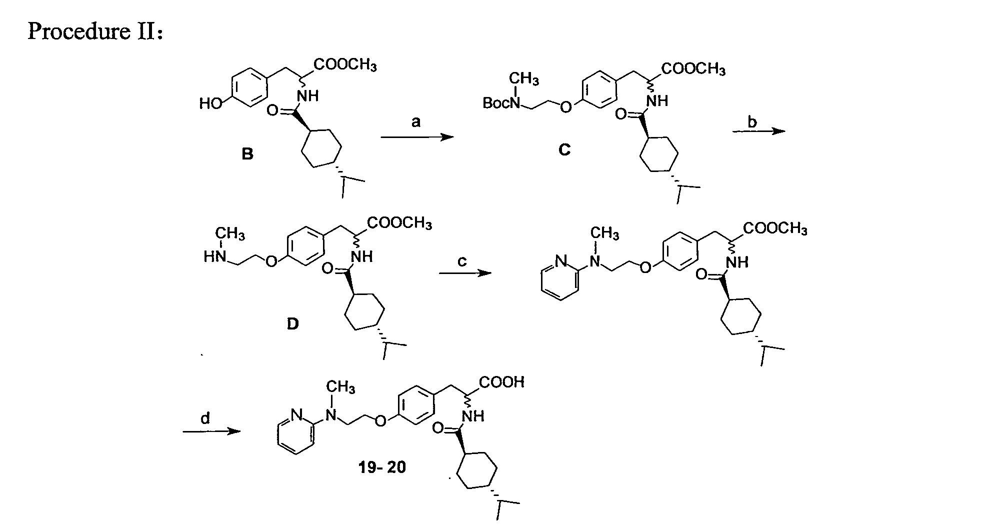

In the scheme: a. triphenylphosphine, diethyl azodicarboxylate, 2-(N-carbobenzoxy-N-methylamino)ethanol, tetrahydrofuran; b. trifluoracetic acid, dichloromethane; c. 2-fluoropyridine, reflux; d. 1N lithium hydroxide, tetrahydrofuran/methanol (3:1).

An embodiment of procedure II is as follows:
1. Compound B is prepared by the same method as in Procedure I.
2. Compound B condenses with *tert*-butoxycarbonyl protecting 2-methylaminoethanol to obtain (2*S*)-2-[N-(*trans*-4-isopropylcyclohexylcarbonyl) amino]-3-[4-(N-methyl-N-*tert*-butoxycarbonylaminoethoxy)phenyl]propionic acid methyl ester (compound C). The solvent used in condensation reaction is the anhydrous inert solvent such as anhydrous tetrahydrofuran, anhydrous ether, chloroform, dichloromethane or the like. The reaction temperature is between 0-100 °C. The reaction time is between 0.1-3 days. The compound C is treated with trifluoracetic acid under -10-50°C for 1-72h to remove the protective group and obtain (2*S*)-2-[N-(*trans*-4-isopropylcyclohexylcarbonyl)amino]-3-[4-(N-methylaminoethoxy)phenyl]propionic acid methyl ester (compound D). Then compound D is refluxed and condensed with excessive 2-fluoropyridine to obtain the ester precursors of compounds 19-20. Then compounds 19-20 are obtained by hydrolyzation with base. The suitable inorganic bases for hydrolyzation are sodium hydroxide, lithium hydroxide, potassium hydroxide, sodium carbonate, potassium carbonate, lithium carbonate, etc. The hydrolyzing temperatures is between -10-100°C. The hydrolyzing solvent is a mixture of tetrahydrofuran and methanol in different ratios, or a mixture of other inert solvents, such as chloroform, dichloromethane, benzene, and suitable alcohols, such as ethanol, propanol, isopropanol in different ratios. The optimal hydrolyzing condition is to hydrolyze with lithium hydroxide under room temperature for 24h with tetrahydrofuran-methanol (3:1) as solvent.
3. If desired corresponding pharmaceutical acceptable salts are prepared.

The representative alanine compounds of formula (I) according to the present invention are listed as follows:
(1) (2*S*)-2-[N-(*trans*-4-isopropylcyclohexylcarbonyl)amino]-3-[4-[2-(5-methyl-2-phenyl-4-oxazolyl) ethoxy]phenyl]propionic acid;
(2) (2*S*)-2-[N-(*trans*-4-isopropylcyclohexylcarbonyl)amino]-3-[4-[2-[N-methyl-N-(2-benzoxazolyl)amino]ethoxy]phenyl]propionic acid (3);
(3) (2*R*)-2-[N-(trans-4-isopropylcyclohexylcarbonyl)amino]-3-[4-[2-(5-methyl-2-phenyl-4-oxazolyl)ethoxy]phenyl]propionic acid (2);
(4) (2*R*)-2-[N-(*trans*-4-isopropylcyclohexylcarbonyl)amino]-3-[4-[2-[N-methyl-N-(2-benzoxazolyl)amino]ethoxy]phenyl]propionic acid;
(5) (2*S*)-2-[N-(*trans*-4-isopropylcyclohexylcarbonyl)amino]-3-[4-[2-(1-indolyl) ethoxy]phenyl]propionic acid;
(6) (2*R*)-2-[N-(*trans*-4-isopropylcyclohexylcarbonyl)amino]-3-[4-[2-(1-indolyl) ethoxy]phenyl]propionic acid;
(7) (2*S*)-2-[N-(*trans*-4-isopropylcyclohexylcarbonyl)amino]-3-[4-(4-trifluoromethylbenzy loxy)phenyl]propionic acid;
(8) (2*R*)-2-[N-(*trans*-4-isopropylcyclohexylcarbonyl)amino]-3-[4-(4-trifluoromethylbenz yloxy)phenyl]propionic acid;
(9) (2*S*)-2-[N-(*trans*-4-isopropylcyclohexylcarbonyl)amino]-3-(4-benzyloxyphen yl) propionic acid;
(10) (2*R*)-2-[N-(*trans*-4-isopropylcyclohexylcarbonyl)amino]-3-(4-benzyloxyphe nyl) propionic acid;
(11)(2*S*)-2-[N-(*trans*-4-isopropylcyclohexylcarbonyl)amino]-3-(4-butoxyphenyl) propionic acid;
(12)(2*R*)-2-[N-(*trans*-4-isopropylcyclohexylcarbonyl)amino]-3-(4-butoxyphenyl) propionic acid;
(13) (2*S*)-2-[N-(*trans*-4-isopropylcyclohexylcarbonyl)amino]-3-(4-ethoxyphenyl) propionic acid;
(14)(2*R*)-2-[N-(*trans*-4-isopropylcyclohexylcarbonyl)amino]-3-(4-ethoxyphenyl) propionic acid;
(15)(2*S*)-2-[N-(*trans*-4-isopropylcyclohexylcarbonyl)amino]-3-(4-methoxyphenyl) propionic acid;
(16)(2*R*)-2-[N-(*trans*-4-isopropylcyclohexylcarbonyl)amino]-3-(4-ethoxyphenyl) propionic acid;
(17)(2*S*)-2-[N-(*trans*-4-isopropylcyclohexylcarbonyl)amino]-3-(4-hydroxyphenyl) propionic acid;
(18)(2*R*)-2-[N-(*trans*-4-isopropylcyclohexylcarbonyl)amino]-3-(4-hydroxyphenyl) propionic acid;
(19)(2*S*)-2-[N-(*trans*-4-isopropylcyclohexylcarbonyl)amino]-3-[4-[2-[N-methyl-N-(2-pyridyl)amino]ethoxyl]phenyl]propionic acid;
(20)(2R)-2-[N-(trans-4-isopropylcyclohexylcarbonyl)amino]-3-[4-[2-[N-methyl-N-(2-pyridyl)amino]ethoxyl]phenyl]propionic acid;
(21)(2*S*)-2-[N-(*trans*-4-isopropylcyclohexylcarbonyl)amino]-3-(4-hydroxyphenyl) propionic acid methyl ester;
   or
(22)(2*R*)-2-[N-(*trans*-4-isopropylcyclohexylcarbonyl)amino]-3-(4-hydroxyphenyl) propionic acid methyl ester.
The structure formulas of compounds 1-20 mentioned above are listed in table 1. Table 1:

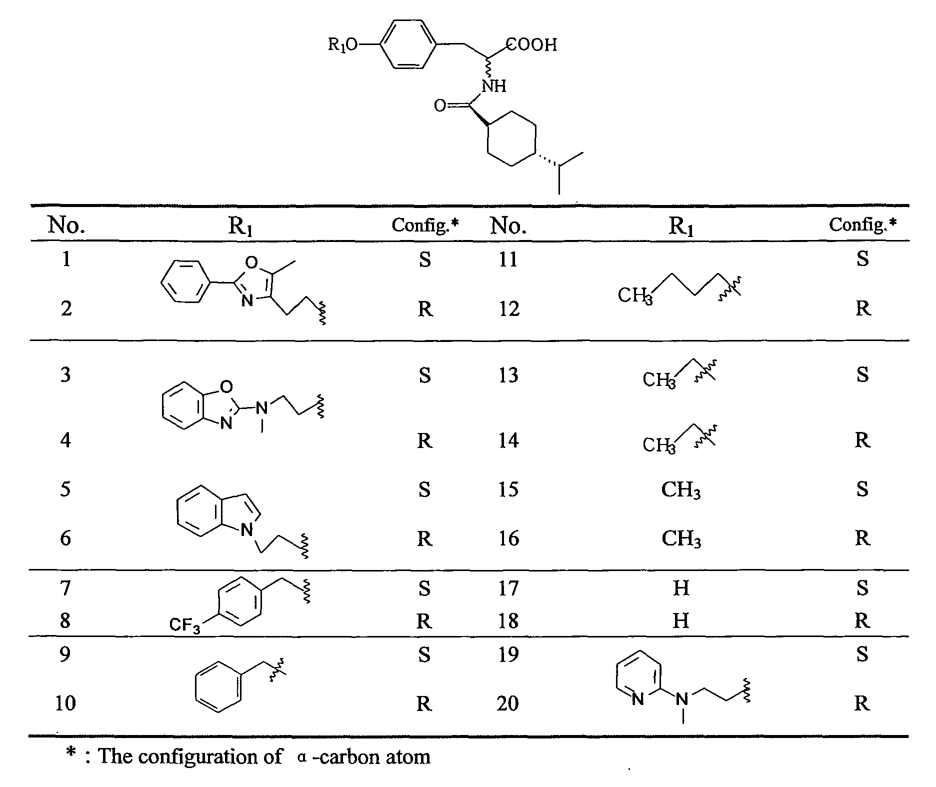

### Evaluation of biological activities:

Insulin-sensitizing agents can promote the differentiation of preadipocytes toward adipocytes, thus insulin-sensitizing agents could be identified according to the differentiation of pre-adipocytes. Following the methods reported in reference [Kletzein BF. Mol. Pharm. 1991, 41, 393], the insulin-sensitizing activities of the compounds of formula (I) in the present invention were evaluated in 3T3-L1 preadipocyte model with triglyceride accumulation in cells as the indication of differentiation.

The 3T3-L1 preadipocytes were incubated in DMEM (Dulbecco's Modified Eagle's Medium) containing 10% NBS (newborn calf serum) and subcultured every 3 days. The cells were transferred to 24-pore plates, and after the pores were filled completely the cells were treated with IBMX (isobutylmethylxanthine) (0.5mmol/L), DEX (dexamethasone) (1µmol/L) and insulin (1.0µmol/L) for 48h, and in the meantime different amounts of test compounds were added and the cells were incubated until the end of the experiment. Cells were collected and the contents of triglyceride and protein therein were determined by colorimetry, and the enhancements of triglyceride in cells after drug-administrating were calculated.

The positive control group is rosiglitazone, the solvent control group is culturing medium containing 0.1% of DMSO. The enhancement of triglyceride in cells was determined in three different concentrations (0.01, 0.1, 1 µmol/L) of the tested compounds.

The insulin-sensitizing activity of the alanine compounds in the present invention are presented in table 2, from which it can be seen that Compound 1 and compound 2 have stronger insulin-sensitizing activity. Thus the alanine compounds in the present invention can be used to control the blood glucose level in type II diabetes patients and inhibit the occurrence of complications.

The alanine compounds in the present invention don't contain the thiazolidinedione group, but possess similar insulin-sensitizing activity as the thiazolidinedione compounds. Therefore, it is possible that these compounds are developed as novel drugs for treatment of type II diabetes and its complications.

**Table 2.**

| The Percentage of Triglyceride Increased in 3T3-L1 Cells | | | |
|---|---|---|---|
| Compounds | Concentrations^{a} (µmol/L) | | |
| | 0.01 | 0.1 | 1 |
| **1** | 1. 56 | 21. 18 | 47.09 |
| **2** | 10. 73 | 16.54 | 30.68 |
| **3** | -0.37 | 6.70 | 19.97 |
| **4** | -4.77 | 5.05 | 10.97 |
| **5** | -1.54 | -4.64 | -6.92 |
| **6** | ND | ND | ND |
| **7** | 11.75 | 5.80 | 4.68 |
| **8** | ND | ND | ND |
| **9** | 6.00 | 3.35 | -0.69 |
| **10** | ND | ND | ND |
| **11** | 0.00 | 0.36 | 2. 23 |
| **12** | ND | ND | ND |
| **13** | 1. 89 | 1. 83 | -4. 74 |
| **14** | ND | ND | ND |
| **15** | -0. 21 | 5. 99 | 5. 04 |
| **16** | ND | ND | ND |
| **17** | 0.22 | 10.84 | 0.85 |
| **18** | ND | ND | ND |
| **19** | -1.46 | -2.24 | -1.62 |
| Rosiglitazone ^{b} | 27.20±2.35 | 34.93±2.14 | 39.21±2.27 |

| | | | |
|---|---|---|---|
| ^{a} Average data in 3 pores. ^{b} Sample number n=22; mean± SE. ND: Not Done. | | | |

The compounds and their pharmaceutical acceptable salts of the present invention may be prepared into many forms of preparations, which contain a safe and effective dosage of the compounds or their pharmaceutical acceptable salts in the present invention, and the pharmaceutical acceptable carrier.

"safe and effective dosage" means the amount of the compounds that can improve the condition of patients but do not lead to serious side effects. The safe and effective dosage of compounds is determined according to the age, condition, and course of treatment of the subjects accepting the therapy.

"Pharmaceutical acceptable carrier" refers to one or many kinds of compatible solid or liquid stuffing materials or gel substances, which are suitable for man use and have enough purity and low toxicity. " compatible " refers to each component in the compositions can mixed with the compounds of the present invention and with each other without significantly reducing the effect of the compounds. Examples of the pharmaceutical acceptable carrier include cellulose and its derivatives (CMC-Na, EC-Na, cellulose acetatic acid ester etc.), gelatin, steatite, solid lubricants (such as stearic acid, magnesium stearate), CaSO₄, vegetable oils (such as soya oil, sesame oil, peanut oil, olive oil etc.), polybasic alcohol (such as propylene glycol, glycerin, mannitol, sorbierite etc.), emulsifying agent (such as tweens®), moistening agent (such as sodium dodecylsulfate), coloring agent, flavoring agent, stabilizer, antioxidant, antiseptic, pyrogen-free water etc.

### The Preferable Embodiments of the Invention

The present invention will be further explained with reference to the following examples, but they don't limit the present invention in any way. In all examples, the melting points were measured with MEL-TEMP melting point apparatus and the thermometer was not calibrated; ¹H NMR spectra were recorded on a Varian Mercury 400 NMR spectrometer, the chemical shifts are expressed as δ (ppm); silica gel for separation is 200-300 mesh unless otherwise specified.

### Example 1: (2S)-2-[N-(trans-4-isopropylcyclohexylcarbonyl)amino]-3-[4-[2-(5-methyl-2-phenyl-4-oxazole)ethoxy]phenyl]propionic acid(1);

(1) condensation: 0.605g(2.31mmol) of triphenylphosphine was added into a solution of 0.313g (1.54mmol) of 2-(5-methyl-2-phenyl-4-oxazole)ethanol and 0.535g (1.54mmol) of (2*S*)-2-[N-(*trans*-4-isopropylcyclohexylcarbonyl)amino]-3-(4-hydroxyphenyl)propionic acid methyl ester ((*S*)-B) in 30ml of anhydrous tetrahydrofuran, then 370µl (2.31mmol) of diethyl azodicarboxylate was added dropwise at 0°C. The resulting solution was stirred at room temperature for 24h. After solvent was removed under reduced pressure, the residue was diluted with ether and a solid separated out, the white solid was filtered out and recrystallized with methanol to give 0.44g of (2*S*)-2-[N-(trans-4-isopropylcyclohexylcarbonyl)amino] -3-[4-[2-(5-methyl- 2-phenyl-4-oxazole)ethoxy]phenyl] propionic acid methyl ester, yield: 53.8%.
(2) hydrolysis: 0.26g (0.5mmol) of the resultant in step (1) was dissolved in 1ml of mixed solvent of tetrahydrofuran- methanol (3:1), then 1ml of 1N solution of LiOH in H₂O was added, the resulting solution was stirred at room temperature for 24h. The pH of the solution was adjusted to 5 with 1N HCl, and 5mL of H₂O was added, then this mixture was stirred and filtered, the filter cake was recrystallizated with methanol to give 0.2g of compound (1), yield: 77.2%. m.p. 151-153°C(dec). [α]_{D}²⁵ 81.1(c, 0.545, CHCl₃).
   ¹H NMR (DMSO-d₆): δ = 0.80(d, J=7.6Hz, 6H, isoproply-CH₃), 0.85-1.0(m, 3H, isoproply-CH, cyclohexyl-CH₂-), 1.10-1.40(m, 3H, cyclohexyl-CH₂-, -CH-), 1.50-1.72(m, 4H, cyclohexyl-CH₂-), 2.00(m, 1H, cyclohexyl-CH-), 2.35(s, 3H, oxazolyl-CH₃), 2.75(dd, J=14Hz, 9.9Hz, 1H, Ph-CH-), 2.90(t, J=6.6Hz, 2H, oxazolyl-CH₂-), 2.96(dd, J=14Hz, 4.7Hz, 1H, Ph-CH-), 4.17(t, J=6.6Hz, 2H, -CH₂O-), 4.21(m, 1H, -CHCOO-), 6.81(d, J=8.8Hz, 2H, Ph-H), 7.13(d, J=8.8Hz, 2H, Ph-H), 7.50(m, 3H, Ph-H), 7.92(m, 3H, Ph-H, -NH), 12.6(s, 1H, -COOH); ¹³C NMR (CDCl₃): δ = 10.0, 19.9, 25.8, 28.8, 29.2, 29.5, 32.6, 36.1, 43.0, 45.9, 53.0, 66.2, 114.3, 126.2, 127.0, 128.0, 128.5, 130.3, 130.4, 132.1, 145.9, 157.8, 160.0, 175.8, 176.3.
   Elemental Analysis, C₃₁H₃₈N₂O₅ (518):
   Calculated C, 71.81; H, 7.34; N, 5.41.
   Found C, 71.49; H, 7.24; N, 5.36.
   IR (KBr): 3282.3, 2933.2, 1710.6, 1631.5, 1554.4, 1513.9, 1251.6, 684.6 cm⁻¹; EI-MS(m/z): 518(1, M⁺), 186(100); HRMS: 518.2772 (C₃₁H₃₈N₂O₅).

### Example 2: (2R)-2-[N-(trans-4-isopropylcyclohexylcarbonyl)amino]-3-[4-[2-(5-methyl-2-phenyl-4-oxazolyl)ethoxy]phenyl]propionic acid(2)

With (2*R*)-2-[N-(trans-4-isopropylcyclohexylcarbonyl)amino]-3-(4-hydroxyphenyl)propionic acid methyl ester ((*R*)-B) as starting material, compound (2) was prepared by the same method as in example 1. m.p. 151-153 °C (dec). [α]_{D}²⁵ -82.5 (c, 0.217, CHCl₃). ¹H NMR is the same as that of compound (1) in example 1.
Elemental Analysis, C₃₁H₃₈N₂O₅(518):
Calculated C, 71.81; H, 7.34; N, 5.41.
Found C, 71.40; H, 8.16; N, 5.33.
IR(KBr): 3282.3, 2933.2, 2854.2, 1712.5, 1633.4, 1554.4, 1513.9, 1249.7,1176.4, 715.5, 686.5 cm⁻¹.

### Example 3: (2S)-2-[N-(trans-4-isopropyleyclohexylcarbonyl)amino]-3-[4-[2-[N-methyl-N-(2-benzoxazolyl)amino]ethoxy]phenyl]propionic acid (3)

A solution of 11g (0.21mmol) of compound D in 2ml of tetrahydrofuran was added with 240µl (0.51mmol) of triethylamine and 40mg (0.26mmol) of 2-chlorobenzoxazole. The resulting solution was stirred at room temperature for 24h. Tetrahydrofuran was removed under reduced pressure, the residue was mixed with 4ml of ethyl acetate, then added 4ml of saturated solution of NaHCO₃ in H₂O and stirred. Remained standing and separated the ethyl acetate layer, dried with anhydrous Na₂SO₄, and the residue was mixed with a mixed solvent of petrolether/ethyl acetate (1:1) to separate out a white solid. The solid was hydrolyzed with LiOH to give 0.042g of the target compound. Yield: 39.6%. m.p. 179-180°C(dec). [α]_{D}²⁵ 81.1 (c, 0.535, CHCl₃).
¹H NMR(DMSO): δ =0.81(d, J=6.9Hz, 6H), 0.80-1.0(m, 3H), 1.15-1.40(m, 3H), 1.60(m, 4H), 1.95(m, 1H), 2.78(dd, J=13.5Hz, 7.3Hz, 1H), 2.96(dd, J=13.5Hz, 4.8Hz, 1H), 3.20(s, 3H), 3.82(t, J=5.5Hz, 2H), 4.10(m, 1H), 4.20(t, J=5.5Hz, 2H), 6.78(d, J=8.4Hz, 2H), 6.95(t, J=7.7Hz, 1H), 7.00(d, J=8.4Hz, 2H), 7.10(t, J=7.0Hz, 1H), 7.28(d, J=8.3Hz, 1H), 7.35(d, J=8.0Hz, 1H), 7.40(d, J=7.3Hz, 1H).
Elemental Analysis, C₂₉H₃₇N₃O₅ (507):
Calculated C, 68.64; H, 7.30; N, 8.28.
Found C, 68.45; H, 7.49; N, 8.21;
IR(KBr) : 3322.8, 2935.2, 2865.7, 1731.8, 1648.9, 1589.1, 1513.9, 1465.7, 1247.7, 1250.3, 925.7, 740.5 cm⁻¹; EI-MS(m/z): 507(12, M⁺), 148(100).

### Example 4: (2R)-2-[N-(trans-4-isopropylcyclohexylcarbonyl)amino]-3-[4-[2-[N-methyl-N-(2-benzoxazolyl)amino]ethoxy]phenyl]propionic acid (4);

Using (R)-B as starting material, compound (4) was prepared by the same method in example 3. m.p. 179-180°C. ¹H NMR is the same as that of compound (3) in example 3.
Elemental Analysis, C₂₉H₃₇N₃O₅ (507):
Calculated C, 68.64; H, 7.30; N, 8.28.
Found C, 68.64; H, 7.22; N, 8.08;
EI-MS(m/z): 507(6, M⁺), 148(100).

### Example 5: (2S)-2-[N-(trans-4-isopropylcyclohexylcarbonyl)amino]-3-[4-[2-(1-indolyl) ethoxy]phenyl]propionic acid (5)

Using 2-(1-indolyl)ethanol and compound (*S*)-B as starting materials, following the procedure as in example 1, the residue after removal of tetrahydrofuran was mixed with methanol and then separated out a solid. The target compound was obtained by hydrolyzation of the solid with LiOH. Yield: 39.6%.
¹H NMR(CDCl₃): δ =0.81(d, J=6.9Hz, 6H), 0.80-1.10(m, 3H), 1.30-1.45(m, 3H), 1.70-1.90(m, 4H), 1.99(m, 1H), 3.07(dd, J=14.3Hz, 6.2Hz, 1H), 3.15(dd, J=14.3Hz, 5.2Hz, 1H), 4.23(t, J=5.5Hz, 2H), 4.50(t, J=5.5Hz, 2H), 4.75(m, 1H), 5.89(d, J=6.6Hz, 1H), 6.50(d, J=3.9Hz, 1H), 6.76(d, J=8.4Hz, 2H), 7.00(d, J=8.4Hz, 2H), 7.11(t, J=7.3Hz, 1H), 7.24(m, 2H), 7.40(d, J=8.1Hz, 1H), 7.62(d, J=7.7Hz, 1H);
IR(KBr): 3305.4, 2933.2, 2863.8, 1712.5, 1650.8, 1513.9, 1463.7, 1242.0, 742.5 cm⁻¹; EI-MS(m/z): 476(11, M⁺), 144(100); HRMS: 476.2670 (C₂₉H₃₆N₂O₄).

### Example 6: (2R)-2-[N-(trans-4-isopropylcyclohexylcarbonyl)amino]-3-[4-[2-(1-indolyl)ethoxy]phenyl]propionic acid (6)

using (R)-B as starting materials, compound (6) was prepared following the same procedure as in example 5. ¹H NMR is the same as that of compound (5) in example 5.
IR(KBr): 3291.9, 2933.2, 2863.8, 1712.5, 1650.8, 1513.9, 1463.7, 1242.0, 742.5 cm⁻¹.

### Example 7: (2S)-2-[N-(trans-4-isopropylcyclohexylcarbonyl)amino]-3-[4-(4-trifluoromethylbenzyloxy)phenyl]propionic acid (7)

Using 4-trifluoromethylbenzyl alcohol and compound (*S*)-B as starting materials, ethyl ether as solvent, compound (7) was prepared following the procedure as of compound 21. Yield: 60.6%. m.p. 171-172°C. [α]_{D}²⁵ 53.9 (c, 0.285, CHCl₃).
¹H NMR(CDCl₃): δ =0.81(d, J=6.7Hz, 6H), 1.0(m, 3H), 1.40(m, 3H), 1.70-1.90(m, 4H), 2.05(m, 1H), 3.08(dd, J=5.5Hz, 14.1Hz, 1H), 3.19(dd, J=5.5Hz, 14.3Hz, 1H), 4.80(m, 1H), 5.08(s, 2H), 5.98(d, J=6.4Hz, 1H), 6.88(d, J=8.2Hz, 2H), 7.08(d, J=8.2Hz, 2H), 7.52(d, J=8.0Hz, 2H), 7.62(d, J=8.2Hz, 2H).
Elemental Analysis, C₂₇H₃₂F₃NO₄·1/2H₂O (500):
Calculated C, 64.80; H, 6.60; N, 2.80.
Found C, 65.20; H, 6.43; N, 3.01.
IR(KBr): 3328.6, 2931.3, 1731.8, 1612.2, 1511.9, 1328.7, 1243.9, 1166.7, 1124.3, 1068.4, 1018.2, 827.3 cm⁻¹; EI-MS(m/z): 491(3, M⁺), 159(100).

### Example 8: (2R)-2-[N-(trans-4-isopropylcyclohexylcarbonyl)amino]-3-[4-(4-trifluoromethylbenzyloxy)phenyl]propionic acid (8)

Using (R)-B as starting materials, compound (8) was prepared following the same procedure as in example 7. m.p. 171-172°C. ¹H NMR is the same as that of the compound 7.
EI-MS(m/z): 491(10, M⁺), 159(100); HRMS: 491.2260(C₂₇H₃₂F₃NO₄).

### Example 9:

### (2S)-2-[N-(trans-4-isopropylcyclohexylcarbonyl)amino]-3-(4-benzyloxyphenyl) propionic acid (9)

A solution of 0.137ml (1.14mmol) of benzyl bromide and 0.13g (0.38mmol) of (2*S*)-2-[N-(trans-4-isopropylcyclohexylcarbonyl)amino]-3-(4-hydroxyphenyl)propioni c acid methyl ester (compound (*S*)-B) in lml N,N-dimethylformamide (DMF) was added with 0.16g (1.14mmol) of levigated K₂CO₃. The resulting mixture was stirred at 70°C for 12h. After cooling to 0°C, 5ml of H₂O was added, the resulting solution was extracted with ethyl acetate (5ml×2). The combined extract was washed with H₂O, dried with anhydrous Na₂SO₄. Solvent was removed under reduced pressure, the residue mixed with ether, and a white solid separated. The solid was hydrolyzed with LiOH to give 0.10g of the target compound. Yield: 62.5%. m.p. 140-142°C(dec). [α]_{D}²⁵ 70.9 (c, 0.83, CHCl₃).
¹H NMR(CDCl₃): δ =0.81(d, J=6.9Hz, 6H), 1.0(m, 3H), 1.40(m, 3H), 1.70-1.90(m, 4H), 2.05(m, 1H), 3.04(dd, J=5.5Hz, 13.9Hz, 1H), 3.19(dd, J=5.5Hz, 13.9Hz, 1H), 4.81(m, 1H), 5.01 (s, 2H), 6.12(d, J=7.3Hz, 1H), 6.91(d, J=8.4Hz, 2H), 7.08(d, J=8.4Hz, 2H), 7.39(m, 5H).
Elemental Analysis, C₂₆H₃₃NO₄·1/2H₂O (432):
Calculated C, 72.22; H, 7.87; N, 3.24.
Found C, 72.34; H, 7.71; N, 3.47;
IR(KBr): 3328.6, 2929.4, 2852.2, 1731.8, 1648.9, 1529.3, 1513.9, 1452.2, 1243.9, 1178.3, 1027.9, 732.8, 694.3 cm⁻¹; EI-MS(m/z): 423(4, M⁺), 254(24), 91(100).

### Example 10: (2R)-2-[N-(trans-4-isopropylcyclohexylcarbonyl)amino]-3-(4-benzyloxyphenyl)propionic acid (10)

using (R)-B as starting materials, compound (10) was prepared following the same procedure as in example 9. m.p. 140-142°C(dec). ¹H NMR is the same as that of the compound 9.
Elemental Analysis, C₂₆H₃₃NO₄·1/2H₂O (432):
Calculated C, 72.22; H, 7.87; N, 3.24.
Found C, 72.36; H, 7.91; N, 3.32.
IR(KBr): 3326.7, 2929.4, 2852.2, 1729.9, 1648.9, 1529.3, 1513.9, 1452.2, 1243.9, 1178.3, 1027.9, 732.8, 694.3 cm⁻¹; EI-MS(m/z): 423(12, M⁺), 254(100).

### Example 11: (2S)-2-[N-(trans-4-isopropylcyclohexylcarbonyl)amino]-3-(4-butoxyphenyl)propionic acid (11)

Using n-bromobutane and compound (*S*)-B as starting materials, compound (11) was prepared following the similar synthesis procedure as in example 9. Yield: 70.3 %. m.p. 120-121 °C. [α]_{D}²⁵ 87.9 (c, 1.145, CHCl₃).
¹H NMR(CDCl₃): δ=0.82(d, J=6.9Hz, 6H), 0.96(m, 6H), 1.38(m, 3H), 1.50(m, 2H), 1.75-1.90(m, 6H), 2.05(m, 1H), 3.04(dd, J=5.8Hz, 14.3Hz, 1H), 3.19(dd, J=5.5Hz, 14.2Hz, 1H), 3.91(t, J=6.6Hz, 2H), 4.80(m, 1H), 5.99(d, J=7.3Hz, 1H), 6.81(d, J=8.4Hz, 2H), 7.07(d, J=8.4Hz, 2H).
Elemental Analysis, C₂₃H₃₅NO₄·1/3H₂O (395):
Calculated C, 69.87, H, 9.03, N, 3.54.
Found C, 69.60, H, 8.88, N, 3.64.
IR(KBr): 3305.4, 2933.2, 2869.6, 1716.4, 1646.9, 1544.7, 1513.9, 1245.8, 1178.3, 827.3 cm⁻¹; EI-MS(m/z): 389(7, M⁺), 163(100).

### Example 12: (2R)-2-[N-(trans-4-isopropylcyclohexylcarbonyl)amino]-3-(4-butoxyphenyl)propionic acid (12)

Using n-bromobutane and compound (R)-B as starting materials, compound (12) was prepared following the similar synthesis procedure as in example 9. m.p. 120-121 °C. ¹H NMR is the same as that of the compound 11.
Elemental Analysis, C₂₃H₃₅NO₄ (389):
Calculated C, 70.95; H, 9.00; N,3.60.
Found C, 71.05; H, 9.11; N, 3.93.
IR(KBr): 3320.9, 2869.6, 2933.2, 1731.8, 1652.7, 1612.2, 1544.7, 1511.9, 1243.9, 827.3 cm⁻¹; EI-MS(m/z): 389(7, M⁺), 163(67), 220(100).

### Example 13: (2S)-2-[N-(trans-4-isopropylcyclohexylcarbonyl)amino]-3-(4-ethoxyphenyl)propionic acid (13)

Using ethyl bromide and compound (*S*)-B as starting materials, compound (13) was prepared following the procedure as in example 9. Yield: 75.0%. m.p. 168-170 °C. [α]_{D}²⁵ 93.2 (c, 1.13, CHCl₃).
¹H NMR(CDCl₃): δ =0.81(d, J=6.9Hz, 4H), 1.0(m, 3H), 1.40(m, 5H), 1.75-1.90(m, 4H), 2.05(m, 1H), 3.04(dd, J=5.5Hz, 14.0Hz, 1H), 3.17(dd, J=5.1Hz, 14.3Hz, 1H), 4.00(m, 2H), 4.80(m, 1H), 5.97(d, J=7.6Hz, 1H), 6.81(d, J=8.4Hz, 2H), 7.03(d, J=8.3Hz, 2H).
ElementalAnalysis, C₂₁H₃₁NO₄·1/3H₂O (367):
Calculated C, 68.66; H, 8.63; N, 3.81.
Found C, 68.93; H, 8.43; N, 3.86.
IR(KBr): 3303.5, 2935.2, 2958, 1724.1, 1708.6, 1643.1, 1542.8, 1513.9, 1442.5, 1247.7, 1215, 1176.4, 1051, 525.4 cm⁻¹; EI-MS(m/z): 361(16, M⁺), 192(100).

### Example 14: (2R)-2-[N-(trans-4-isopropylcyclohexylcarbonyl)amino]-3-(4-ethoxyphenyl)propionic acid (14)

Using ethyl bromide and compound (R)-B as starting materials, compound (14) was prepared following the synthesis procedure in example 9. m.p. 168-170°C. ¹H NMR is the same as that of the compound 13.
Elemental Analysis, C₂₁H₃₁NO₄ (361):
Calculated C, 69.81; H, 8.59; N, 3.87.
Found C, 69.40; H, 8.32; N, 3.90.
IR(KBr): 3305.4, 2933.2, 1706.7, 1641.2, 1540.9, 1513.9, 1444.4, 1249.7, 1215, 1049.1, 925.7, 804.2 cm⁻¹.

### Example 15: (2S)-2-[N-(trans-4-isopropylcyclohexylcarbonyl)amino]-3-(4-methoxyphenyl)propionic acid (15)

Using methyl iodide and compound (*S*)-B as starting materials, compound (15) was prepared following the synthesis procedure in example 9. Yield: 67.1%. m.p. 149-151°C. [α]_{D}²⁵ 96.5 (c, 0.665, CHCl₃).
¹H NMR(CDCl₃): δ = 0.85(d, J=6.6Hz, 6H), 1.00(m, 3H), 1.40(m, 3H), 1.71-1.90(m, 4H), 2.05(m, 1H), 3.10(dd, J=5.9Hz, 14.0Hz, 1H), 3.17(dd, J=5.5Hz, 13.9Hz, 1H), 3.80(s, 3H), 4.80(m, 1H), 5.98(d, J=7.6Hz, 1H), 6.81(d, J=8.8Hz, 2H), 7.03(d, J=8.8Hz, 2H).
Elemental Analysis, C₂₀H₂₉NO₄ (347):
Calculated C, 69.16; H, 8.36; N, 4.03.
Found C, 69.06; H, 8.54; N, 4.14.
IR (KBr): 3280.4, 2937.1, 2860, 1720.2, 1646.9, 1542.8, 1515.8, 1440.6, 1249.7, 1215, 1031.7, 829.3 cm⁻¹; EI-MS(m/z): 347(1, M⁺), 121(100).

### Example 16:

### (2R)-2[N-(trans-4-isopropylcyclohexylcarbonyl)amino]-3-(4-ethoxyphenyl)propio nic acid (16)

Using methyl iodide and compound (R)-B as starting materials, compound (16) was prepared following the similar synthesis procedure in example 9. m.p. 149-151°C. ¹H NMR is the same as that of compound (*S*)-15.
Elemental Analysis, C₂₀H₂₉NO₄·1/3H₂O(353):
Calculated C, 67.99; H, 8.41; N, 3.97.
Found C, 68.44; H, 8.08; N, 4.07.
IR (KBr): 3274.6, 2939, 2860, 1720, 1645, 1552.4, 1513.9, 1440, 1249.7, 1216.9, 1031.7, 829.3 cm⁻¹; EI-MS(m/z): 347(16, M⁺), 121(100).

### Example 17: (2S)-2-[N-(trans-4-isopropylcyclohexylcarbonyl)amino]-3-(4-hydroxyphenyl)propionic acid (17)

The target compound was obtained by hydrolysis of (*S*)-B with LiOH. Yield: 81.1 %. m.p. 156-157°C. [α]_{D}²⁵ 76.4 (c, 0.845, CHCl₃).
¹H NMR(DMCO-d6): δ =0.81(d, J=7.0Hz, 6H), 1.0(m, 3H), 1.40(m, 3H), 1.75-1.90(m, 4H), 2.15(m, 1H), 2.90(dd, J=8.1Hz, 13.9Hz, 1H), 3.10(dd, J=5.1Hz, 13.9Hz, 1H), 4.82(m, 1H), 6.72(d, J=8.4Hz, 2H), 7.05(d, J=8.4Hz, 2H).
Elements Analysis, C₁₉H₂₇NO₄·2H₂O(369):
Calculated C, 61.79; H, 8.40; N, 3.79.
Found C, 61.94; H, 8.17; N, 3.84.
IR(KBr): 3303.5, 2937.1, 2861.9, 1619.9, 1546.7, 1517.7, 1446.4, 1232.3, 827.3 cm⁻¹; EI-MS(m/z): 333(10, M⁺), 170(100), 107(84).

### Example 18: (2R)-2-[N-(trans-4-isopropylcyclohexylcarbonyl)amino]-3-(4-hydroxyphenyl)propionic acid (18)

The compound (18) was obtained by hydrolysis of (R)-B with LiOH. m.p. 156-157°C. ¹H NMR is the same as that of compound 17.
Elemental Analysis, C₁₉H₂₇NO₄·2/3H₂O(345):
Calculated C, 66.09; H, 8.21; N, 4.06.
Found C, 65.57; H, 8.71; N, 4.03.
IR(KBr): 3309.3, 2939.0, 2861.9, 1745.3, 1726.0, 1619.9, 1517.7, 1548.6, 1444.4, 1230.4, 825.4, 680.8, 538.1 cm⁻¹; EI-MS(m/z): 333(10, M⁺), 170(100), 107(54).

### Example 19: (2S)-2-[N-(trans-4-isopropylcyclohexylcarbonyl)amino]-3-[4-[2-[N-methyl-N-(2-pyridyl)amino]ethoxyl]phenyl]propionic acid (19)

A solution of 0.19g (1.1mmol) of 2-[(N-*tert*-butoxycarbonyl)-methylamino]ethanol and 0.347g (1mmol) of (*S*)-B in 15ml of anhydrous tetrahydrofuran was added with 0.38g (1.5mmol) of triphenylphosphine, then 240µl (1.5mmol) of diethyl azodicarboxylate was added drop-wise at 0°C. The resulting solution was stirred at room temperature for 24h.The solvent was removed under reduced pressure, and the residue was chromatographed over silica gel H column with petroleum/ethyl acetate (2: 1) as eluent to obtain 0.21 g of a colorless syrup, yield: 41.6 %.

0.21g (0.42mmol) of the colorless syrup obtained in last step was dissolved in 4.8ml of dichloromethane, then 4.8ml of trifluoroacetic acid was added. The resulting solution was stirred at room temperature for 1h. Then a part of the solvent was removed under reduced pressure at room temperature. The residual solution was neutralized with saturated NaHCO₃ and extracted with dichloromethane (10ml×2). The combined organics were washed with H₂O, dried on Mg₂SO₄, filtered and concentrated in vacuo. The solvent was evaporated off from the filtrate. The residue was refluxed with 2ml of 2-fluoropyridine for 24h. Excessive 2-fluoropyridine was removed under reduced pressure, the residue was dissolved in small amount of acetone, then chromatographed over silica gel H column with acetone/petroleum (1:2) as eluent to obtain 0.081g of white solid. Hydrolysis of the white solid following the same procedure in example 1 provided 0.06g of compound (19). Yield: 76.8%. m.p. 158-160°C. [α]_{D}²⁵30.0 (c, 0.26, CHCl₃).
¹H NMR (CDCl₃): δ = 0.80 (d, J=7.6Hz, 6H), 0.80-1.00(m, 3H), 1.13-1.40(m, 3H), 1.62-1.82(m, 4H), 1.96(m, 1H), 3.03(d, J=4.4Hz, 2H), 3.10(s, 3H), 3.88(t, J=6.5Hz), 3.98(t, J=6.6Hz), 4.62(m, 1H), 5.40(s, 1H), 6.21(d, J=6.9Hz, 1H), 6.55(m, 1H), 6.75(d, J=8.5Hz, 2H), 6.98(d, J=8.5Hz, 2H), 7.48(m, 1H), 8.03(m, 1H).
Elemental Analysis, C₂₇H₃₇N₃O₄·1/2H₂O(476):
Calculated C, 68.07; H, 7.98; N, 8.82.
Found C, 67.86; H, 7.75; N, 8.79.
IR (KBr): 3299.7, 2931.3, 1720.2, 1637.3, 1608.4, 1511.9, 1425.2, 1247.7, 1213, 1000.9, 763.7 cm⁻¹; EI-MS(m/z): 467(2, M⁺), 121(98), 135(100).

### Example 20: (2R)-2-[N-(trans-4-isopropylcyclohexylcarbonyl)amino]-3-[4-[2-[N-methyl-N-(2-pyridyl)amino]ethoxyl]phenyl]propionic acid (20)

Compound (20) was obtained by the same procedure as in example 19 using (R)-B as starting material. m.p. 154-155°C. [α]_{D}²⁵ -30.5(c, 0.19, CHCl₃). ¹H NMR is the same as that of compound 19. IR (KBr): 3315.1, 2921.7, 2854.2, 1594.9, 1502.3, 1421.3, 1247.7, 765.6 cm⁻¹.

### Example 21: (2S)-2-[N-(trans-4-isopropylcyclohexylcarbonyl)amino]-3-(4-hydroxyphenyl) propionic acid methyl ester ((S)-B)

A solution of 0.7g (4.1mmol) of *trans*-4-isopropylcyclohexylcarboxylic acid and 0.53g (4.6mmol) of HOSu in 14ml of chloroform was added 0.95g (4.6mmol)of DCC batchwise. The resulting solution was stirred at room temperature for 3h. The solid produced was filtered off. 0.4ml of glacial acetic acid was added into the filtrate and stirred at room temperature. After 2h, 10ml of saturated aqueous solution of NaHCO₃ was added into, stirred, and the water layer was removed. The chloroform layer was washed with 5ml of H₂O and saturated saline solution, then dried over anhydrous Mg₂SO₄ and filtered under reduced pressure. 0.80g (4.1mmol) of L-tyrosine methyl ester was added, then stirred at room temperature for 24h. The solution was washed with 1N HCl and water, dried with anhydrous Mg₂SO₄ and filtered under reduced pressure. After removal of the solvent under reduced pressure, the residue was dissolved in methanol, stand at -20°C, a white solid was separated out. The mother liquor was concentrated and then another portion of solid was collected. 0.53g of white compound ((*S*)-B) was obtained together. Yield: 37.8 %.
¹H NMR(CDCl₃): δ =0.82(d, J=6.9Hz, 6H), 0.89-1.12(m, 3H), 1.30-1.43(m, 3H), 1.70-1.90(m, 4H), 2.05(m, 1H), 2.96(dd, J=6.5Hz, 14.2Hz, 1H), 3.09(dd, J=5.5Hz, 14.7Hz, 1H), 3.72(s, 3H), 4.85(m, 1H), 6.00(d, J=8.1Hz, 1H), 6.70(d, J=8.4Hz, 2H), 6.90(d, J=8.2Hz, 2H).

### Example 22: (2R)-2-[N-(trans-4-isopropylcyclohexylcarbonyl)amino]-3-(4-hydroxyphenyl) propionic acid methyl ester ((R)-B)

(R)-B was obtained with D-tyrosine methyl ester as starting material by following the above operations.

## Claims

1. Alanine compounds represented by following formula (I) or their salts: 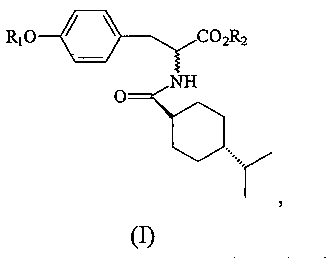 wherein the configuration of the α-carbon atom of alanine is R or S;
R₁ is hydrogen, substituted or unsubstituted C₁₋₆ alkyl, or aromatic ring or aromatic heterocyclic ring group selected from the following group: 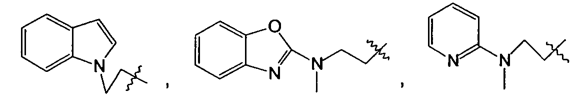 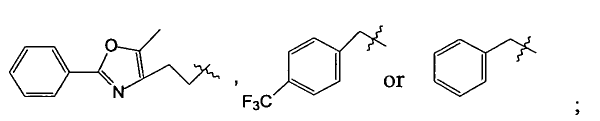 R₂ is hydrogen or substituted or unsubstituted C₁₋₆ alkyl.

2. The alanine compounds or their salts of claim 1 which are selected from the group consisting of:
(1) (2*S*)-2-[N-(*trans*-4-isopropylcyclohexylcarbonyl)amino]-3-[4-[2-(5-methyl-2 -phenyl-4-oxazolyl) ethoxy]phenyl]propionic acid;
(2) (2*S*)-2-[N-(*trans*-4-isopropylcyclohexylcarbonyl)amino]-3-[4-[2-[N-methyl-N-(2-benzoxazolyl)amino]ethoxy]phenyl]propionic acid;
(3) (2*R*)-2-[N-(trans-4-isopropylcyclohexylcarbonyl)amino]-3-[4-[2-(5-methyl-2-phenyl-4-oxazolyl)ethoxy]phenyl]propionic acid;
(4) (2*R*)-2-[N-(*trans*-4-isopropylcyclohexylcarbonyl)amino]-3-[4-[2-[N-methyl-N-(2-ben zoxazolyl)amino]ethoxy]phenyl]propionic acid;
(5) (2*S*)-2-[N-(*trans*-4-isopropylcyclohexylcarbonyl)amino]-3-[4-[2-(1-indolyl)-ethoxy]phenyl]propionic acid;
(6) (2*R*)-2-[N-(*trans*-4-isopropylcyclohexylcarbonyl)amino]-3-[4-[2-(1-indolyl)-ethoxy]phenyl]propionic acid;
(7) (2*S*)-2-[N-(*trans*-4-isopropylcyclohexylcarbonyl)amino]-3-[4-(4-trifluoromethyl-benz yloxy)phenyl]propionic acid;
(8) (2*R*)-2-[N-(*trans*-4-isopropylcyclohexylcarbonyl)amino]-3-[4-(4-trifluoromethylbenz yloxy)phenyl]propionic acid;
(9) (2*S*)-2-[N-(*trans*-4-isopropylcyclohexylcarbonyl)amino]-3-(4-benzyloxy phenyl) propionic acid;
(10) (2*R*)-2-[N-(*trans*-4-isopropylcyclohexylcarbonyl)amino]-3-(4-benzyloxyphenyl) propionic acid;
(11)(2*S*)-2-[N-(*trans*-4-isopropylcyclohexylcarbonyl)amino]-3-(4-butoxyphenyl)-propionic acid;
(12)(2*R*)-2-[N-(*trans*-4-isopropylcyclohexylcarbonyl)amino]-3-(4-butoxyphenyl)-propionic acid;
(13) (2*S*)-2-[N-(*trans*-4-isopropylcyclohexylcarbonyl)amino]-3-(4-ethoxyphenyl)-propionic acid;
(14)(2*R*)-2-[N-(*trans*-4-isopropylcyclohexylcarbonyl)amino]-3-(4-ethoxyphenyl)-propionic acid;
(15)(2*S*)-2-[N-(*trans*-4-isopropylcyclohexylcarbonyl)amino]-3-(4-methoxyphenyl)propionic acid;
(16)(2*R*)-2-[N-(*trans*-4-isopropylcyclohexylcarbonyl)amino]-3-(4-ethoxyphenyl)p ropionic acid;
(17)(2*S*)-2-[N-(*trans*-4-isopropylcyclohexylcarbonyl)amino]-3-(4-hydroxyphenyl) propionic acid;
(18)(2*R*)-2-[N-(*trans*-4-isopropylcyclohexylcarbonyl)amino]-3-(4-hydroxyphenyl) propionic acid;
(19) (2*S*)-2-[N-(*trans*-4-isopropylcyclohexylcarbonyl)amino]-3-[4-[2-[N-methyl-N-(2-pyridyl)amino]ethoxyl]phenyl]propionic acid;
(20) (2R)-2-[N-(trans-4-isopropylcyclohexylcarbonyl)amino]-3-[4-[2-[N-methyl-N-(2-pyridyl)amino]ethoxyl]phenyl]propionic acid;
(21) (2*S*)-2-[N-(*trans*-4-isopropylcyclohexylcarbonyl)amino]-3-(4-hydroxyphenyl) propionic acid methyl ester; or
(22) (2*R*)-2-[N-(*trans*-4-isopropylcyclohexylcarbonyl)amino]-3-(4-hydroxyphenyl) propionic acid methyl ester.

3. A method of preparing the alanine compounds or their salts of claim 1, comprising the following steps:
(1) *trans*-4-isopropylcyclohexylcarboxylic acid N-hydroxylsuccinimide ester and L-tyrosine methyl ester or D-tyrosine methyl ester conduct a condensation in inert solvents to produce 2-[N-(*trans*-4-isopropylcyclohexylcarbonyl) amino]-3-(4-hydroxyphenyl) propionic acid methyl ester;
(2) 2-[N-(*trans*-4-isopropylcyclohexylcarbonyl)amino]-3-(4-hydroxyphenyl) propionic acid methyl ester and the corresponding heterocycloalkyl alcohol or aromatic alcohol conduct Mitsunobu reaction, followed by hydrolyzing with an inorganic base to obtain the compounds of formula (I), wherein R₁ is 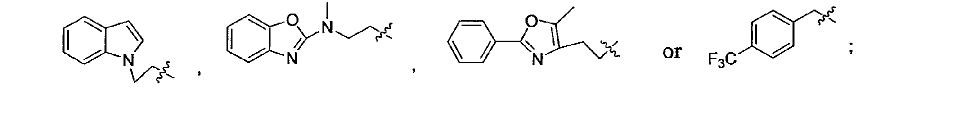 R₂ is hydrogen; or
2-[N-(*trans*-4-isopropylcyclohexylcarbonyl)amino]-3-(4-hydroxyphenyl) propionic acid methyl ester and corresponding alkyl halide conduct etherification under basic condition to obtain the compounds of formula (I), wherein R₁ is 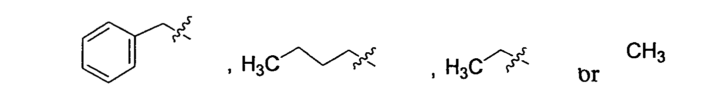 R₂ is hydrogen; or
2-[N-(*trans*-4-isopropylcyclohexylcarbonyl)amino]-3-(4-hydroxyphenyl) propionic acid methyl ester is hydrolyzed to obtain the compound of formula(I), wherein R₁ and R₂ both are hydrogen;
(3) if desired, corresponding pharmaceutical acceptable salts are prepared.

4. The method of claim 3, wherein the inert solvent is selected from chloroform, dichloromethane, ether, tetrahydrofuran.

5. The method of claim 3, wherein the inorganic base for hydrolysis is selected from sodium hydroxide, lithium hydroxide, potassium hydroxide, sodium carbonate, potassium carbonate and lithium carbonate and the solvent for hydrolysis is selected from mixed solvent of tetrahydrofuran and methanol, or mixed alcohol, or chloroform, benzene.

6. The method of claim 3, wherein the inorganic base for etherification is selected from sodium hydroxide, lithium hydroxide, potassium hydroxide, sodium carbonate, potassium carbonate and lithium carbonate and the temperature of etherification is between -10-180°C, suitable solvent is selected from N,N-dimethylformamide, DMSO and H₂O, and the reaction time is 1-72h.

7. A method of preparing the alanine compounds or their salts of claim 1, comprising the following steps:
(1) Condensing 2-[N-(*trans*-4-isopropylcyclohexylcarbonyl)]-3-(4-hydroxyphenyl) propionic acid methyl ester with amino-protected 2-methylaminoethanol, then deprotecting, refluxing with excessive 2-fluoropyridine, and obtaining the compounds of formula (I) by hydrolysis with bases, wherein R₁ is 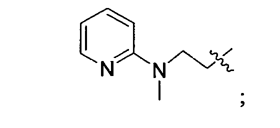 R₂ is hydrogen;
(2) if desired, corresponding pharmaceutical acceptable salts are prepared.

8. The method of claim 7, wherein the inorganic base for hydrolysis is selected from sodium hydroxide, lithium hydroxide, potassium hydroxide, sodium carbonate, potassium carbonate, lithium carbonate and the solvent for hydrolysis is selected from mixed solvent of tetrahydrofuran and methanol, or mixed alcohols, or chloroform, benzene.

9. The use of the alanine compounds of formula (I) and their salts in the preparation of drugs for type II diabetes.
